# EUROPÄISCHE PATENTSCHRIFT

(11) **EP 3 914 577 B1**
(45) Veröffentlichungstag und Bekanntmachung des Hinweises auf die Patenterteilung: **25.09.2024**
(21) Anmeldenummer: 19702364.1
(22) Anmeldetag: 23.01.2019
(51) Int. Cl.: C07C 29/78, C07C 31/20

(54) **VERFAHREN ZUR HERSTELLUNG VON 1,2-ALKANDIOLEN IN FESTER DARREICHUNGSFORM**
METHOD FOR PRODUCING 1,2-ALKANE DIOLS IN A SOLID DOSAGE FORM
PROCÉDÉ DE PRÉPARATION DE 1,2 ALCANEDIOLS SOUS UNE FORME GALÉNIQUE SOLIDE

(43) Veröffentlichungstag der Anmeldung: 01.12.2021
(73) Patentinhaber: Symrise AG, 37603 Holzminden Niedersachsen (DE)
(72) Erfinder: SIEWERT, Jürgen, 37434 Rollshausen (DE); STEPHAN, Torsten, 37603 Holzminden (DE)
(74) Vertreter: Fabry, Bernd
(86) Internationale Anmeldenummer: PCT/EP2019/051575
(87) Internationale Veröffentlichungsnummer: WO 2020/151812

(56) Entgegenhaltungen:
- EP-A1- 1 876 162
- EP-B1- 1 876 162
- WO-A2-2008/046796
- JP-B2- 4 855 217
- US-A- 3 632 657

## Beschreibung

### GEBIET DER ERFINDUNG

Die Erfindung befindet sich auf dem Gebiet der Pastillierungstechnologie und betrifft ein verbessertes Verfahren zur Herstellung von 1,2-Alkandiolen in fester Darreichungsform.

### TECHNOLOGISCHER HINTERGRUND

1,2-Alkandiole werden in den letzten Jahren verstärkt als emulsionsbildende Inhaltsstoffe in verschiedensten Anwendungen eingesetzt - die Anwendungen reichen von Körperpflegemitteln bis hin zu technischen Anwendungen im Bereich Druckertinten.

Während die niederen 1,2-Alkandiole (1,2-Propandiol bis 1,2-Hexandiol) bei Raumtemperatur einen flüssigen Aggregatzustand aufweisen - sind die mittleren bis höheren 1,2-Alkandiole (ab C8 = 1,2-Octandiol) bei Raumtemperatur Feststoffe. Die Schmelzpunkte der Feststoffe steigen mit zunehmender Kettenlänge an:
1,2-Octandiol: 27 bis 31°C
1,2-Decandiol: 45 bis 48°C
1,2-Dodecandiol: 56 bis 60°C
1,2-Tetradecandiol: 64 bis 70°C

Bei den Schmelzen von reinen C₈ bis C₁₄ Alkandiolen (> 95% Gehalt an Alkandiol) beobachtet man nach Abkühlung nur eine geringe Erstarrungsneigung. Die Materialien scheinen für Stunden oder Tage in einem metastabilen, flüssigen Zustand zu verweilen bis sich Keimzellen für die Erstarrung bzw. Kristallisation ausgebildet haben. Nach Entstehen solcher Keimzellen erfolgt anschließend eine Kristallisation welche, je nach Temperaturgradienten, durch ungeordnete Erstarrung der Schmelze überlagert wird. Nach Verfestigung des Produktes erhält man somit ein teilkristallines Produkt.

Der Erstarrungsvorgang der Materialien verläuft üblicherweise unter Volumeneffekten ab - d.h. es erfolgt eine Volumenausdehnung des Materials, wobei das feste Material eine raue, unebene Oberfläche aufweist. Aus produktionstechnischer Sicht ist diese Materialeigenschaft problematisch, da Abfüllgebinde erst nach erfolgter Erstarrung verschlossen werden können, um Deformation der Packmittel zu vermeiden. Um Verzögerungen im Materialfluss zu verringern bzw. zu vermeiden sind schnell erstarrende Schmelzen wünschenswert.

Der Einsatz bzw. die Dosierung von Feststoffen für die Zubereitung von Anwendungsmischungen stellt üblicherweise einen größeren technischen und logistischen Aufwand dar. Die Feststoffe müssen in den Anlieferungsgebinden (typischerweise bis zu 180 kg in Stahlfässern) aufgeschmolzen werden um dann als Flüssigkeit in die Mischung dosiert zu werden. Der Aufschmelzprozess muss hierbei unter einer Inertgasatmosphäre erfolgen, da bei Einwirkung von Luftsauerstoff bei erhöhten Temperaturen eine Produktschädigung nicht ausgeschlossen werden kann. Ein solcher Aufschmelzprozess kann je nach technischen Möglichkeiten des Anwenders bis zu mehreren Stunden oder sogar Tagen dauern. Somit ist eine kurzfristige Produktionsplanung nur eingeschränkt möglich. Weiterhin muss für den Abkühlungsprozess ebenfalls eine Inertgasatmosphäre gewährleistet sein, um Produktschädigungen auszuschließen. Dies ist insbesondere zu beachten wenn nur geringe Mengen an Alkandiolen für eine Mischung eingesetzt werden und die Vorratsgebinde entsprechend häufig aufgewärmt/abgekühlt werden müssen.

### RELEVANTER STAND DER TECHNIK

Aus der WO 2016/016154 A1 (SYMRISE) ist ein Verfahren zur Herstellung von festen Kühlstoffen bekannt, bei dem man eine vorgekrätzte Schmelze von Mentholverbindungen durch gleichmäßige Vertropfung auf eine vorgekühlte Fläche aufgibt.

WO2008046796 A2 beschreibt C10-C14-Alkan-1,2-diole für kosmetische oder dermatologische Anwendungen.

JP4855217 B2 betrifft die Herstellung einer gut handhabbaren 1,2-Octandiol-Formulierung zur kosmetischen Verwendung.

### AUFGBE DER ERFINDUNG

Aus der oben beschriebenen Problematik bei der Herstellung von Mischungen unter Verwendung von geringen Mengen an Alkandiolen ergibt sich die Notwendigkeit einer festen Darreichungsform für 1,2-Alkandiole welche eine Dosierung ohne vorheriges Aufschmelzen zulässt.

Durch die oben beschriebenen Stoffcharakteristiken ist eine direkte Verfestigung der Schmelze von mittleren 1,2-Alkandiolen durch Vertropfen oder Pastillieren, die Herstellung von Schuppen (über eine Schuppenwalze) oder eine Kristallisation nicht möglich. Bei der Vertropfung von z.B. 1,2-Decandiol konnten keine Pastillen, sondern nur ein dünner Produktfilm erhalten werden. Das feste 1,2-Decandiol lag nach Aufgabe der Schmelze als Tropfen auf eine gekühlte Oberfläche nach 2 Minuten vor. Die Ablösung von der gekühlten Oberfläche gestaltete sich schwierig - das verfestigte/geschuppte Material konnte nur unter Bildung von Feinanteilen (Bruchstücken) erhalten werden. Weiterhin unterlag der wachsartige Anteil des erhaltenen Feststoffes einer Nachkristallisation, wobei nach einigen Tagen eine raue, brüchige Oberfläche auf dem Feststoff zutage trat. Bei Lagerung von so erhaltenen Schuppen entsteht durch mechanische Reibung ein nicht zu vernachlässigender Feinanteil im Packmittel bzw. im Falle einer noch nicht vollständig abgeschlossenen Nachkristallisation führt diese zum Verklumpen des Materials. Durch die eben beschriebenen Prozesse wird die Ausbildung sowohl von Fein- als auch von Grobanteilen begünstigt-diese sind für eine spätere Feststoffdosierung unerwünscht und nachteilhaft. Verklumpte Aggregate müssen teilweise unter hohen mechanischen Aufwand vor dem Einsatz wieder vereinzelt werden, während Feinanteile nur unter erhöhtem Schutzaufwand gehandhabt werden können.

Wie vorhergehend beschrieben ist der durch direkte Verfestigung/Vertropfung erhaltene Feststoff von 1,2-Alkandiolen nur von eingeschränktem praktischem Nutzen. Entsprechend ergibt sich die Notwendigkeit von alternativen Herstellverfahren für feste Darreichungsformen.

Die Aufgabe der vorliegenden Erfindung hat daher darin bestanden, ein verbessertes Verfahren zur Herstellung fester Darreichungsformen von 1,2-Alkandiolen zur Verfügung zu stellen, das frei von den eingangs geschilderten Nachteilen ist. Insbesondere sollte sich das Verfahren dadurch auszeichnen, dass die Pastillen einen sehr geringen Feinstaubanteil besitzen, auch nach längerer Lagerung eine möglichst glatte Oberfläche aufweisen und keine Neigung zum Verklumpen zeigen.

### BESCHREIBUNG DER ERFINDUNG

Ein erster Gegenstand der Erfindung betrifft ein Verfahren zur Herstellung von 1,2-Alkandiolen in fester Darreichungsform, umfassend oder bestehend aus den folgenden Schritten:
(a) Bereitstellen einer Schmelze mindestens eines 1,2-Alkandiols;
(b) Abkühlen der Schmelze aus Schritt (a) auf eine Temperatur unterhalb des Schmelzpunktes 1,2-Alkandiols oder der 1,2-Alkandiolmischung unter Erhalt einer vorgekrätzten Schmelze bestehend aus einem unterkühlten 1,2-Alkandiol oder einer unterkühlten 1,2-Alkandiolmischung mit einem Gehalt an 1,2-Alkandiolimpfkristallen;
(c) Inkontaktbringen der Mischung aus Schritt (b) mit einer gekühlten Fläche unter Erhalt von 1,2-Alkandiolen oder 1,2-Alkandiolmischungen in kristalliner Form, mit der Maßgabe, dass man 1,2-Alkandiole einsetzt, die 8 bis 14 Kohlenstoffatome aufweisen.

Auf der Suche nach Problemlösungen wurde die Vertropfung/Pastillierung von vorgekrätzten Schmelzen der 1,2-Alkandiole untersucht. Die Schmelzen wurden hierzu in einem Doppelmantelgefäß mit einem randgängigen Flügelrührer unter Rühren abgekühlt. Hierbei wurden durch den randgängigen Rührer Impfkristalle erzeugt und gleichzeitig zerkleinert. Durch Abkühlung mit einem Thermostaten unter den Schmelzpunkt des 1,2-Alkandiols konnte so eine Mischung/Slurry/Brei von Impfkristallen und dem unterkühlten Diol erzeugt werden. Überraschenderweise konnten solche Mischungen einfach zu Pastillen vertropft werden. Hierzu wurde der Kristallbrei mit einer Pipette auf eine gekühlte Oberfläche getropft - die so erhaltenen Pastillen wurden für eine gewisse Zeit bei Raumtemperatur nachgekühlt und konnten dann mit einem Schaber leicht von der gekühlten Oberfläche entfernt werden.

Die so erhaltenen Pastillen weisen - auch nach längerer Lagerung-eine glatte bis leicht raue Oberfläche auf. Je höher der Kristallanteil in der verarbeiteten Slurry desto glatter erscheinen die Oberflächen. Auch nach längerer Lagerung der frisch hergestellten Pastillen wurde kein Verklumpen der Pastillen beobachtet.

### 1,2-ALKANDIOLE

Erfindungsgemäß werden 1,2-Alkandiole eingesetzt, die 8 bis 14 Kohlenstoffatome aufweisen. Dazu setzt man mindestens ein 1,2-Alkandiol und vorzugsweise mindestens zwei Alkandiole ein, die ausgewählt sind aus der Gruppe, die gebildet wird von 1,2 Octandiol, 1,2-Decandiol, 1,2-Dodecandiol und 1,2-Tetradecandiol. Üblicherweise werden 1,2-Alkandiole in technischer Qualität eingesetzt, die einen 1,2-Alkandiolgehalt von mindestens 92 Gew.-%, vorzugsweise mindestens 95 Gew.-% und insbesondere mindestens 98 Gew.-% aufweisen.

Grundsätzlich ist es auch möglich, den genannten mittelkettigen 1,2-Alkandiolen kürzerkettige Homologe, insbesondere 1,2-Pentaniol und/oder 1,2-Hexandiol zuzusetzen, wobei die Mengen etwa 5 bis etwa 15 Gew.-% betragen können und nach oben hin nur durch eine ausreichende Festigkeit der Endprodukte begrenzt wird.

### PASTILLIERUNGSVERFAHREN

Das erfindungsgemäße Verfahren zeichnet sich insbesondere dadurch aus, dass man 1,2-Alkandiole mit glatter oder annähernd glatter Oberfläche herstellt.

Im ersten Schritt werden die 1,2-Alkandiole aufgeschmolzen, d.h. je nach Kettenlänge auf etwa 60 bis etwa 80 °C erwärmt. Die so erhaltenen Schmelzen werden dann unter Rühren abgekühlt. Dies kann beispielsweise in einem Rührgefäß mit Mantelkühlung unter Einsatz eines Flügelrührers erfolgen. Es wird - wieder je nach Kettenlänge - bei etwa 40 bis 65 °C eine unterkühlte ("vorgekrätzte") Schmelze erhalten, in der sich Impfkristalle bilden. Der Abkühlvorgang wird fortgesetzt, bis der Gehalt an Impfkristallen mindestens 1 Gew.-% und vorzugsweise mindestens 5 Gew.-% beträgt. Hilfsweise kann man auch Impfkristalle von außen zudosieren.

Ein weiterer limitierender Faktor ist die Viskosität, die über das Drehmoment des Rührers bestimmt wird und vorzugsweise im Bereich von etwa 5 bis etwa 15 Ncm und insbesondere etwa 8 bis etwa 12 Ncm liegen sollte.

Hat die vorgekrätzte Schmelze die erforderliche Viskosität erreicht wird sie beispielsweise auf eine gekühlte Fläche vertropft, wobei sich gleichförmige und regelmäßige Pastillen bilden. Nach einer Ruhezeit, die bei Umgebungstemperatur durchgeführt werden und etwa 1 bis etwa 5 Minuten betragen kann, können die Pastillen beispielsweise mit einem Schaber von der gekühlten Fläche abgenommen werden.

Alternativ kann die vorgekrätzte Schmelze auch zwischen zwei gekühlten Flächen verfestigt werden, die vorzugsweise einen Abstand von wenigen Zentimeter bis einigen Millimeter aufweisen können. Hierbei werden feste Massen, aber keine Pastillen erhalten.

Die Temperatur der gekühlten Flächen kann jeweils im Bereich von etwa 15 bis etwa 22 °C und vorzugsweise zwischen 18 und 20 °C liegen.

### 1,2-ALKANDIOLE IN PASTILLIERTER FORM:

Weitere Gegenstände der vorliegenden Erfindung umfassen:
1,2-Decandiol in pastillierter Form, dadurch erhalten, dass man
   (a) 1,2-Decandiol bei etwa 60 °C aufschmilzt,
   (b) die Schmelze aus Schritt (a) unter Rühren schrittweise bis auf etwa 40 bis 43 °C abkühlt, bis die Schmelze Impfkristalle ausscheidet und ein Drehmoment bzw. eine Viskosität von etwa 8 bis etwa 12 Ncm erreicht hat,
   (c) die vorgekrätzte Schmelze aus Schritt (b) auf eine vorgekühlte Fläche vertropft, die eine Temperatur im Bereich von etwa 18 bis etwa 20 °C aufweist und
   (d) die so erhaltenen Pastillen nach einer Verfestigungszeit von etwa 1 bis etwa 5 Minuten von der vorgekühlten Fläche abnimmt.
1,2-Dodecandiol in pastillierter Form, dadurch erhalten, dass man
   (a) 1,2-Dodecandiol bei etwa 60 °C aufschmilzt,
   (b) die Schmelze aus Schritt (a) unter Rühren schrittweise bis auf etwa 50 bis 52 °C abkühlt, bis die Schmelze Impfkristalle ausscheidet und ein Drehmoment bzw. eine Viskosität von etwa 8 bis etwa 12 Ncm erreicht hat,
   (c) die vorgekrätzte Schmelze aus Schritt (b) auf eine vorgekühlte Fläche vertropft, die eine Temperatur im Bereich von etwa 18 bis etwa 20 °C aufweist und
   (d) die so erhaltenen Pastillen nach einer Verfestigungszeit von etwa 1 bis etwa 5
1,2-Tetradecandiol in pastillierter Form, dadurch erhalten, dass man
   (a) 1,2-Tetradecandiol bei etwa 80 °C aufschmilzt,
   (b) die Schmelze aus Schritt (a) unter Rühren schrittweise bis auf etwa 61 bis 63 °C abkühlt, bis die Schmelze Impfkristalle ausscheidet und ein Drehmoment bzw. eine Viskosität von etwa 8 bis etwa 12 Ncm erreicht hat,
   (c) die vorgekrätzte Schmelze aus Schritt (b) auf eine vorgekühlte Fläche vertropft, die eine Temperatur im Bereich von etwa 18 bis etwa 20 °C aufweist und
   (d) die so erhaltenen Pastillen nach einer Verfestigungszeit von etwa 1 bis etwa 5 Minuten von der vorgekühlten Fläche abnimmt.

### GEWERBLICHE ANWENDBARKEIT

Die nach dem erfindungsgemäßen Verfahren erhältlichen festen 1,2-Alkandiole können beispielsweise in Säcke abgepackt und praktisch beliebig lange gelagert werden, ohne dass es zu Verklumpen oder Abrieb und Bildung von Feinstaub kommt.

Die Pastillen können für vielfältige Zwecke genutzt werden, beispielsweise als Bestandteile von kosmetischen oder pharmazeutischen Zubereitungen.

### BEISPIELE

### Beispiel 1

### Decandiol-1,2

### Herstellung der Krätze:

In einem thermostatisierten 250 mL Doppelmantelgefäß mit einem randgängigen Blattrührer wurde eine Schmelze (60°C) von 1,2-Decandiol (98%) bei konstanter Rührgeschwindikeit (50 U/min) schrittweise abgekühlt. (Das vom Rührer aufzuwendende Drehmoment wurde als Maß für die Viskosität der Schmelze erfasst.) Nach Erreichen des Schmelzpunktes konnten erste Kristalle in der Schmelze beobachtet werden. Optional können zu diesem Zeitpunkt Impfkristalle als Starthilfe zugegeben werden um die Ausbildung des Kristallbettes zu beschleunigen. Die Temperatur der Krätzmenge wurde weiter um 0,2°C/h abgekühlt wobei eine Zunahme der Kristallmenge beobachtet werden konnte. Wurde die gewünschte Viskosität der Schmelze erreicht wurden mit Hilfe einer Pipette Tropfen auf eine auf 18-20°C gekühlte Stahloberfläche gegeben. Die Dauer bis zur Verfestigung der Pastillen wurde bestimmt.

### b) Ergebnisse der Pastillierung:

| | |
|---|---|
| Temperatur der Schmelze [°C] | 42,8 - 41,7 |
| Drehmoment (Viskosität) [Ncm ] | 8,2 - 11,7 |
| Verfestigungsdauer [sec ] | 10-30 |
| Schmelzpunkt [°C ] | 47,1-47,6 |
| Durchschnittliche Höhe [ mm] | 3,5 - 5,5 |
| Durchmesser [mm ] | 11- 16 |
| Einzelgewicht [g ] | 0,3 - 0,5 |

Nach einer Zeit von 1 min wurden die so erhaltenen Pastillen mit Hilfe eines Schabers von der Metalloberfläche entfernt und die Konsistenz der Pastille geprüft. Die Pastillen waren zu diesem Zeitpunkt vollständig erstarrt.

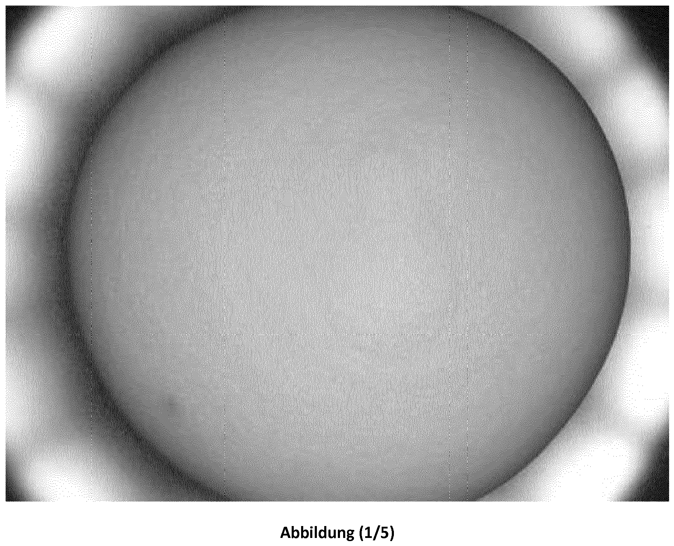

Für Lagertests wurden Pastillen nach der Entnahme von der Metalloberfläche in einer Schütthöhe von 20 cm über 4 Wochen bei Raumtemperatur gelagert. Nach dieser Zeit wurde das Lagergefäß auf Verklumpung und Feinanteile überprüft. Die gemäß Beispiel 1.1 erzeugten Pastillen waren schüttfahig und wiesen einen sehr geringen Verklumpungsgrad auf. Feinanteile waren in dem Lagergefäß nicht zu beobachten. **Abbildung 1** zeigt eine Pastille nach Beispiel 1.

### Beispiel 2

### Doppelseitige Kühlung

Eine nach obigem Verfahren hergestellte Slurry mit einer Temperatur von 42,2°C (9,5 Ncm) wurde zwischen zwei gleichmäßig beabstandete, gekühlte Metallflächen (18°C, Distanz 2-5 mm) gegeben. Nach einer Kontaktzeit von 1 min wurde die obere Fläche entfernt und der erhaltene Feststoff wurde mittels eines Spatels zu Schuppen zerbrochen. Nach Entfernung von Feinanteilen wurde das Material direkt zu Lagertests eingesetzt. Nach einer Lagerzeit von 4 Wochen wurde das Lagergefäß auf Verklumpung und Feinanteile überprüft. Die gelagerten Schuppen waren leicht zu vereinzeln und wiesen einen geringen Feinanteil auf (bedingt durch mechanische Belastung bei der Befüllung).

### Vergleichsbeispiel V1

Schmelzen von Decandiol-1,2 (98,5%) wurden mit einer definierten Temperatur mittels einer Pipette auf eine temperierte Stahloberfläche (19-21°C) vertropft. Die benötigte Zeit bis zur Erstarrung der so erzeugten Tropfen/Schuppen wurde bestimmt.

### b) Ergebnisse der Pastillierung:

| | |
|---|---|
| Temperatur der Schmelze 45°C/50°C/60°C | 1,5 - 2,0 - 2,5 |
| Schmelzpunkt [°C ] | 47,1-47,6 |
| Durchschnittliche Höhe [ mm] | 3,0 - 3,5 |
| Durchmesser [mm ] | 9 - 11 |
| Einzelgewicht [g ] | 0,2 - 0,3 |

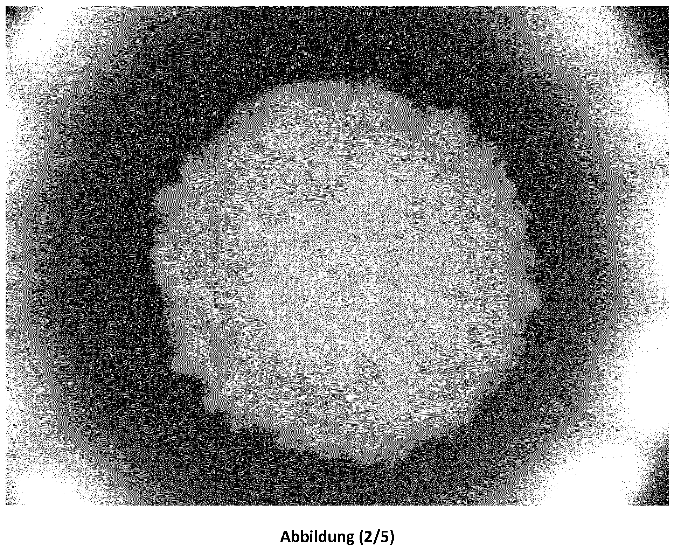

Die auf die gekühlte Oberfläche aufgesetzten Tropfen zerliefen stärker, wodurch breitere, flächigere und wenig erhabene Feststoffe erhalten wurden. (geringere Höhe, mehr Grundfläche). In allen Fällen wurden Feststoffe erhalten, welche eine raue, unebene Oberfläche mit blasenartigen Einschlüssen aufwiesen. Für Lagertests wurden Pastillen nach der Entnahme von der Metalloberfläche in einer Schütthöhe von ca. 20 cm über 4 Wochen bei Raumtemperatur gelagert. Nach dieser Zeit wurde das Lagergefäß auf Verklumpung und Feinanteile überprüft. Die gemäß Beispiel 2 erzeugten Pastillen waren nicht schüttfahig und wiesen einen hohen Verklumpungsgrad auf. Feinanteile, bedingt durch die filigrane, raue Oberfläche, waren in dem Lagergefäß vorhanden. Diese Feinanteile tragen zusätzlich zu der rauen Oberfläche der Pastillen zur Bildung von Agglomeraten/zur Verklumpung der Pastillen bei. **Abbildung 2** zeigt eine Pastille gemäß dem Vergleichsbeispiel V1.

### Beispiel 3

### Dodecandiol-1,2

### a) Herstellung der Krätze

In einem thermostatisierten 250 mL Doppelmantelgefäß mit einem randgängigen Blattrührer wurde eine Schmelze (60°C) von 1,2-Dodecandiol (99%) bei konstanter Rührgeschwindigkeit (50 U/min) schrittweise abgekühlt. (Das vom Rührer aufzuwendende Drehmoment wurde als Maß für die Viskosität der Schmelze erfasst.) Nach Erreichen des Schmelzpunktes konnten erste Kristalle in der Schmelze beobachtet werden. Optional können zu diesem Zeitpunkt Impfkristalle als Starthilfe zugegeben werden um die Ausbildung des Kristallbettes zu beschleunigen. Die Temperatur der Krätzmenge wurde weiter um 0,2°C/h abgekühlt wobei eine Zunahme der Kristallmenge beobachtet werden konnte. Wurde die gewünschte Viskosität der Schmelze erreicht wurden mit Hilfe einer Pipette Tropfen auf eine auf 18-20°C gekühlte Stahloberfläche gegeben. Die Dauer bis zur Verfestigung der Pastillen wurde bestimmt.

### b) Ergebnisse der Pastillierung:

| | |
|---|---|
| Temperatur der Schmelze [°C ] | 52,0 - 51,5 |
| Drehmoment (Viskosität) [Ncm ] | 8,8 - 9,8 |
| Verfestigungsdauer [sec ] | 20 - 30 |
| Schmelzpunkt [°C ] | 59,1-59,5 |
| Durchschnittliche Höhe [ mm] | 3,5 - 5,0 |
| Durchmesser [mm ] | 10 - 15 |
| Einzelgewicht [g ] | 0,3 - 0,4 |

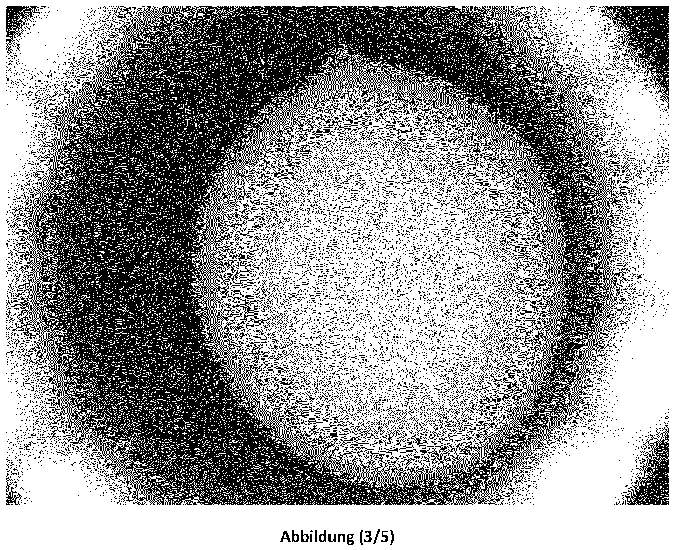

Nach einer Zeit von 1 min wurden die so erhaltenen Pastillen mit Hilfe eines Schabers von der Metalloberfläche entfernt und die Konsistenz der Pastille geprüft. Die Pastillen waren zu diesem Zeitpunkt vollständig erstarrt (d.h. sie wiesen keine weichen/nicht erstarrten Anteile auf). **Abbildung** 3 zeigt eine Pastille nach Beispiel 3.

### Vergleichsbeispiel V2

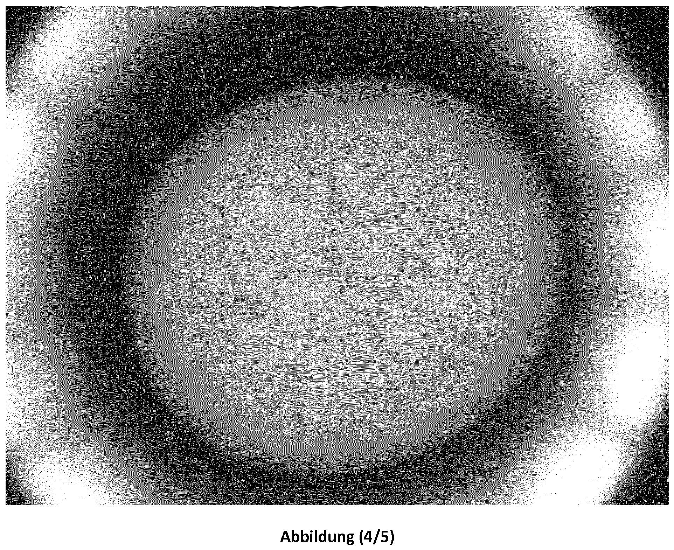

Eine Schmelze von Dodecandiol-1,2 (99%) wurden bei 60°C mittels einer Pipette auf eine temperierte Stahloberfläche (18-20°C) vertropft. Nach einer Zeit von 1 min wurden die Pastillen auf Konsistenz geprüft. Die so erzeugten Pastillen waren nicht vollständig ausgehärtet und wiesen weiche Einschlüsse auf. Die Oberflächen der so erzeugten Pastillen waren rau und uneben. Der Schmelzpunkt der erhaltenen Pastillen: 59,0°C. **Abbildung 4** zeigt eine Pastille gemäß Vergleichsbeispiel V2.

### Beispiel 4

### Herstellung von Tetradecandiol-1,2

### a) Herstellung der Krätze:

In einem thermostatisierten 250 mL Doppelmantelgefäß mit einem randgängigen Blattrührer wurde eine Schmelze (80°C) von 1,2-Tetradecandiol (97%) bei konstanter Rührgeschwindigkeit (50 U/min) schrittweise abgekühlt. (Das vom Rührer aufzuwendende Drehmoment wurde als Maß für die Viskosität der Schmelze erfasst.) Nach Erreichen des Schmelzpunktes konnten erste Kristalle in der Schmelze beobachtet werden. Optional können zu diesem Zeitpunkt Impfkristalle als Starthilfe zugegeben werden um die Ausbildung des Kristallbettes zu beschleunigen. Die Temperatur der Krätzmenge wurde weiter um 0,2°C/h abgekühlt wobei eine Zunahme der Kristallmenge beobachtet werden konnte. Wurde die gewünschte Viskosität der Schmelze erreicht wurden mit Hilfe einer Pipette Tropfen auf eine auf 18-20°C gekühlte Stahloberfläche gegeben. Die Dauer bis zur Verfestigung der Pastillen wurde bestimmt.

### b) Ergebnisse der Pastillierung:

| | |
|---|---|
| Temperatur der Schmelze [°C ] | 62,0 |
| Drehmoment (Viskosität) [Ncm ] | 8,7 - 11,5 |
| Verfestigungsdauer [sec ] | < 10 |
| Schmelzpunkt [°C ] | 66,5 |
| Durchschnittliche Höhe [ mm] | 3,5 - 4,5 |
| Durchmesser [mm ] | 13,5 - 15,5 |
| Einzelgewicht [g ] | 0,5 - 0,6 |

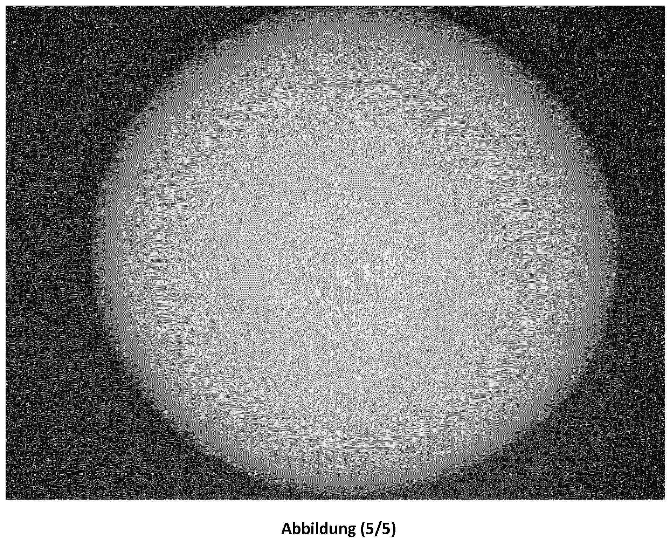

Nach einer Zeit von 1 min wurden die so erhaltenen Pastillen mit Hilfe eines Schabers von der Metalloberfläche entfernt und die Konsistenz der Pastille geprüft. Die Pastillen waren zu diesem Zeitpunkt vollständig erstarrt. **Abbildung 5** zeigt eine Pastille gemäß Beispiel 4.

### Vergleichsbeispiel V3

Eine Schmelze von Tetradecandiol-1,2 (97%) wurden bei 80°C mittels einer Pipette auf eine temperierte Stahloberfläche (18-21°C) vertropft. Nach einer Zeit von 1 min wurden die Pastillen auf Konsistenz geprüft. Die so erzeugten Pastillen waren nicht vollständig ausgehärtet und wiesen weinige weiche Einschlüsse auf. Die Oberflächen der so erzeugten Pastillen waren rau und uneben. Der Schmelzpunkt der erhaltenen Pastillen: 64,3°C

## Patentansprüche

1. Verfahren zur Herstellung von 1,2-Alkandiolen in fester Darreichungsform, umfassend oder bestehend aus den folgenden Schritten:
(a) Bereitstellen einer Schmelze mindestens eines 1,2-Alkandiols;
(b) Abkühlen der Schmelze aus Schritt (a) auf eine Temperatur unterhalb des Schmelzpunktes 1,2-Alkandiols oder der 1,2-Alkandiolmischung unter Erhalt einer vorgekrätzten Schmelze bestehend aus einem unterkühlten 1,2-Alkandiol oder einer unterkühlten 1,2-Alkandiolmischung mit einem Gehalt an 1,2-Alkandiolimpfkristallen;
(c) Inkontaktbringen der Mischung aus Schritt (b) mit einer gekühlten Fläche unter Erhalt von 1,2-Alkandiolen oder 1,2-Alkandiolmischungen in kristalliner Form,
mit der Maßgabe, dass man 1,2- Alkandiole einsetzt, die 8 bis 14 Kohlenstoffatome aufweisen.

2. Verfahren nach 1, **dadurch gekennzeichnet, dass** man mindestens ein 1,2-Alkandiol und vorzugsweise mindestens zwei Alkandiole einsetzt, die ausgewählt sind aus der Gruppe, die gebildet wird von 1,2 Octandiol, 1,2-Decandiol, 1,2-Dodecandiol und 1,2-Tetradecandiol.

3. Verfahren nach mindestens einem der Ansprüche 1 und/oder 2, **dadurch gekennzeichnet, dass** man 1,2-Alkandiole in technischer Qualität einsetzt, die einen 1,2-Alkandiolgehalt von mindestens 92 Gew.-%, vorzugsweise mindestens 95 Gew.-% aufweisen.

4. Verfahren nach mindestens einem der Ansprüche 1 bis 3, **dadurch gekennzeichnet, dass** man 1,2-Alkandiole mit glatter oder annähernd glatter Oberfläche herstellt.

5. Verfahren nach mindestens einem der Ansprüche 1 bis 4, **dadurch gekennzeichnet, dass** man die Schmelzen unter Rühren abkühlt.

6. Verfahren nach mindestens einem der Ansprüche 1 bis 5, **dadurch gekennzeichnet, dass** man die Schmelzen solange abkühlt, bis der Anteil an Impfkristallen in der vorgekrätzten Schmelze mindestens 1 Gew.-% beträgt.

7. Verfahren nach mindestens einem der Ansprüche 1 bis 6, **dadurch gekennzeichnet, dass** man der vorgekrätzten Schmelze zusätzliche Impfkristalle zufügt.

8. Verfahren nach mindestens einem der Ansprüche 1 bis 7, **dadurch gekennzeichnet, dass** man die vorgekrätzte Schmelze abkühlt, bis diese beim Rühren ein Drehmoment bzw. eine Viskosität im Bereich von etwa 5 bis etwa 15 Ncm aufweist.

9. Verfahren nach mindestens einem der Ansprüche 1 bis 8, **dadurch gekennzeichnet, dass** die vorgekrätzten Schmelzen auf eine gekühlte Fläche vertropft werden.

10. Verfahren nach mindestens einem der Ansprüche 1 bis 8, **dadurch gekennzeichnet, dass** die vorgekrätzten Schmelzen zwischen zwei gekühlten Flächen verfestigt werden.

11. Verfahren nach mindestens einem der Ansprüche 1 bis 10, **dadurch gekennzeichnet, dass** die gekühlten Flächen eine Temperatur im Bereich von etwa 15 bis etwa 22 °C aufweisen.

12. 1,2-Decandiol in pastillierter Form, dadurch erhalten, dass man
(a) 1,2-Decandiol bei etwa 60 °C aufschmilzt,
(b) die Schmelze aus Schritt (a) unter Rühren schrittweise bis auf etwa 40 bis 43 °C abkühlt, bis die Schmelze Impfkristalle ausscheidet und ein Drehmoment bzw. eine Viskosität von etwa 8 bis etwa 12 Ncm erreicht hat,
(c) die vorgekrätzte Schmelze aus Schritt (b) auf eine vorgekühlte Fläche vertropft, die eine Temperatur im Bereich von etwa 18 bis etwa 20 °C aufweist und
(d) die so erhaltenen Pastillen nach einer Verfestigungszeit von etwa 1 bis etwa 5 Minuten von der vorgekühlten Fläche abnimmt.

13. 1,2-Dodecandiol in pastillierter Form, dadurch erhalten, dass man
(a) 1,2-Dodecandiol bei etwa 60 °C aufschmilzt,
(b) die Schmelze aus Schritt (a) unter Rühren schrittweise bis auf etwa 50 bis 52 °C abkühlt, bis die Schmelze Impfkristalle ausscheidet und ein Drehmoment bzw. eine Viskosität von etwa 8 bis etwa 12 Ncm erreicht hat,
(c) die vorgekrätzte Schmelze aus Schritt (b) auf eine vorgekühlte Fläche vertropft, die eine Temperatur im Bereich von etwa 18 bis etwa 20 °C aufweist und
(d) die so erhaltenen Pastillen nach einer Verfestigungszeit von etwa 1 bis etwa 5 Minuten von der vorgekühlten Fläche abnimmt.

14. 1,2-Tetradecandiol in pastillierter Form, dadurch erhalten, dass man
(a) 1,2-Tetradecandiol bei etwa 80 °C aufschmilzt,
(b) die Schmelze aus Schritt (a) unter Rühren schrittweise bis auf etwa 61 bis 63 °C abkühlt, bis die Schmelze Impfkristalle ausscheidet und ein Drehmoment bzw. eine Viskosität von etwa 8 bis etwa 12 Ncm erreicht hat,
(c) die vorgekrätzte Schmelze aus Schritt (b) auf eine vorgekühlte Fläche vertropft, die eine Temperatur im Bereich von etwa 18 bis etwa 20 °C aufweist und
(d) die so erhaltenen Pastillen nach einer Verfestigungszeit von etwa 1 bis etwa 5 Minuten von der vorgekühlten Fläche abnimmt.

## Claims

1. A process for the preparation of 1,2-alkanediols in solid dosage form, comprising or consisting of the following steps:
(a) providing a melt of at least one 1,2-alkanediol;
(b) cooling the melt from step (a) to a temperature below the melting point of 1,2-alkanediol or the 1,2-alkanediol mixture to obtain a pre-scratched melt consisting of a supercooled 1,2-alkanediol or a supercooled 1,2-alkanediol mixture containing 1,2-alkanediol seed crystals;
(c) contacting the mixture of step (b) with a cooled surface to obtain 1,2-alkanediols or 1,2-alkanediol mixtures in crystalline form,
with the proviso that 1,2-alkanediols which have 8 to 14 carbon atoms are used.

2. The process according to 1, **characterized in that** at least one 1,2-alkanediol and preferably at least two alkanediols are used, which are selected from the group formed by 1,2-octanediol, 1,2-decanediol, 1,2-dodecanediol and 1,2-tetradecanediol.

3. The process according to at least one of claims 1 and/or 2, **characterized in that** 1,2-alkanediols of technical grade are used which have a 1,2-alkanediol content of at least 92% by weight, preferably at least 95% by weight.

4. The process according to at least one of claims 1 to 3, **characterized in that** 1,2-alkanediols with a smooth or approximately smooth surface are produced.

5. The process according to at least one of claims 1 to 4, **characterized in that** the melts are cooled with stirring.

6. The process according to at least one of claims 1 to 5, **characterized in that** the melts are cooled until the proportion of seed crystals in the pre-scratched melt is at least 1% by weight.

7. The process according to at least one of claims 1 to 6, **characterized in that** additional seed crystals are added to the pre-etched melt.

8. The process according to at least one of claims 1 to 7, **characterized in that** the pre-etched melt is cooled until it exhibits a torque or a viscosity in the range from about 5 to about 15 Ncm during stirring.

9. The process according to at least one of claims 1 to 8, **characterized in that** the pre-scratched melts are dripped onto a cooled surface.

10. The process according to at least one of claims 1 to 8, **characterized in that** the pre-scratched melts are solidified between two cooled surfaces.

11. The process according to at least one of claims 1 to 10, **characterized in that** the cooled surfaces have a temperature in the range from about 15 to about 22°C.

12. 1,2-decanediol in pastillized form, obtained by
(a) melting 1,2-decanediol at about 60 °C,
(b) cooling the melt from step (a) stepwise to about 40 to 43°C with stirring until the melt precipitates seed crystals and has reached a torque or viscosity of about 8 to about 12 Ncm,
(c) dripping the pre-scratched melt from step (b) onto a pre-cooled surface which has a temperature in the range from about 18 to about 20 °C, and
(d) removing the pastilles thus obtained from the pre-cooled surface after a solidification time of about 1 to about 5 minutes.

13. 1,2-dodecanediol in pastillized form, obtained by
(a) melting 1,2-dodecanediol at about 60 °C,
(b) cooling the melt from step (a) stepwise to about 50 to 52°C with stirring until the melt precipitates seed crystals and has reached a moment of rotation or a viscosity of about 8 to about 12 Ncm,
(c) dripping the pre-scratched melt from step (b) onto a pre-cooled surface which has a temperature in the range from about 18 to about 20 °C, and
(d) removing the pastilles thus obtained from the pre-cooled surface after a solidification time of about 1 to about 5 minutes.

14. 1,2-tetradecanediol in pastillized form, obtained by
(a) melting 1,2-tetradecanediol at about 80 °C,
(b) cooling the melt from step (a) stepwise to about 61 to 63°C with stirring until the melt precipitates seed crystals and has reached a moment of rotation or a viscosity of about 8 to about 12 Ncm,
(c) dripping the pre-scratched melt from step (b) onto a pre-cooled surface which has a temperature in the range from about 18 to about 20 °C, and
(d) removing the pastilles thus obtained from the pre-cooled surface after a solidification time of about 1 to about 5 minutes.

## Revendications

1. Procédé de préparation de 1,2-alcanediols sous forme de dosage solide, comprenant ou consistant dans les étapes suivantes :
(a) fournir un fondu d'au moins un 1,2-alcanediol ;
(b) refroidissement de la matière fondue de l'étape (a) à une température inférieure au point de fusion du 1,2-alcanediol ou du mélange de 1,2-alcanediol afin d'obtenir une matière fondue pré-grattée constituée d'un 1,2-alcanediol surfondu ou d'un mélange de 1,2-alcanediol surfondu contenant des cristaux de semences de 1,2-alcanediol ;
(c) mise en contact du mélange de l'étape b) avec une surface refroidie pour obtenir des 1,2-alcanediols ou des mélanges de 1,2-alcanediols sous forme cristalline,
à condition d'utiliser des 1,2-alcanediols ayant de 8 à 14 atomes de carbone.

2. Le procédé selon 1, **caractérisé par** l'utilisation d'au moins un 1,2-alcanediol et de préférence d'au moins deux alcanediols choisis dans le groupe formé par le 1,2-octanediol, le 1,2-décanediol, le 1,2-dodécanediol et le 1,2-tétradécanediol.

3. Le procédé selon l'une au moins des revendications 1 et/ou 2, **caractérisé par** l'utilisation de 1,2-alcanediols de qualité technique dont la teneur en 1,2-alcanediol est d'au moins 92% en poids, de préférence d'au moins 95% en poids.

4. Le procédé selon l'une au moins des revendications 1 à 3, **caractérisé par** la production de 1,2-alcanediols à surface lisse ou à peu près lisse.

5. Le procédé selon l'une au moins des revendications 1 à 4, **caractérisé par le fait que** les produits fondus sont refroidis sous agitation.

6. Le procédé selon l'une au moins des revendications 1 à 5, **caractérisé par le fait que** les matières fondues sont refroidies jusqu'à ce que la proportion de cristaux de semences dans la matière fondue pré-grattée soit d'au moins 1 % en poids.

7. Le procédé selon l'une au moins des revendications 1 à 6, **caractérisé en ce que** des cristaux de semence supplémentaires sont ajoutés à la masse fondue pré-grattée.

8. Le procédé selon l'une au moins des revendications 1 à 7, **caractérisé par le fait que** la matière fondue pré-grattée est refroidie jusqu'à ce qu'elle présente un couple ou une viscosité compris entre environ 5 et environ 15 Ncm lors de l'agitation.

9. Le procédé selon l'une au moins des revendications 1 à 8, **caractérisé par le fait que** les matières fondues pré-grattées sont égouttées sur une surface refroidie.

10. Le procédé selon l'une au moins des revendications 1 à 8, **caractérisé par le fait que** les matières fondues pré-grattées sont solidifiées entre deux surfaces refroidies.

11. Le procédé selon l'une au moins des revendications 1 à 10, **caractérisé par le fait que** les surfaces refroidies ont une température comprise entre 15 et 22°C environ.

12. 1,2-décanediol sous forme pastillée, obtenu par
(a) en faisant fondre le 1,2-décanediol à environ 60 °C,
(b) refroidir la masse fondue de l'étape (a) par étapes jusqu'à environ 40 à 43 °C en agitant jusqu'à ce que la masse fondue précipite des cristaux de semences et atteigne un couple ou une viscosité d'environ 8 à environ 12 Ncm,
(c) égoutter la matière fondue pré-grattée de l'étape (b) sur une surface pré-refroidie dont la température est comprise entre 18 et 20 °C environ, et
(d) retirer les pastilles ainsi obtenues de la surface pré-réfrigéré après un temps de solidification d'environ 1 à 5 minutes.

13. 1,2-dodécanediol sous forme de pastilles, obtenu par
(a) en faisant fondre le 1,2-dodécanediol à environ 60 °C,
(b) refroidir la masse fondue obtenue à l'étape (a) par étapes jusqu'à environ 50 à 52 °C en agitant jusqu'à ce que la masse fondue précipite des cristaux de semences et atteigne un moment de rotation ou une viscosité d'environ 8 à environ 12 Ncm,
(c) égoutter la matière fondue pré-grattée de l'étape (b) sur une surface pré-réfrigéré dont la température est comprise entre 18 et 20 °C environ, et
(d) retirer les pastilles ainsi obtenues de la surface pré-réfrigéré après un temps de solidification d'environ 1 à 5 minutes.

14. 1,2-tetradécanediol sous forme de pastilles, obtenu par
(a) en faisant fondre le 1,2-tetradécanediol à environ 80 °C,
(b) refroidir la masse fondue obtenue à l'étape (a) par étapes jusqu'à environ 61 à 63 °C en agitant jusqu'à ce que la masse fondue précipite des cristaux de semences et atteigne un moment de rotation ou une viscosité d'environ 8 à environ 12 Ncm,
(c) égoutter la matière fondue pré-grattée de l'étape (b) sur une surface pré-réfrigéré dont la température est comprise entre 18 et 20 °C environ, et
(d) retirer les pastilles ainsi obtenues de la surface pré-réfrigéré après un temps de solidification d'environ 1 à 5 minutes.
